# EUROPEAN PATENT SPECIFICATION

(11) **EP 3 319 975 B1**
(45) Date of publication and mention of the grant of the patent: **01.05.2019**
(21) Application number: 16736845.5
(22) Date of filing: 06.07.2016
(51) Int. Cl.: C07H 21/04

(54) **GUANINE-RICH OLIGONUCLEOTIDES**
OLIGONUKLEOTIDE MIT HOHEM GUANINGEHALT
OLIGONUCLÉOTIDES RICHES EN GUANINE

(30) Priority: 08.07.2015 US 201562189832 P; 20.07.2015 EP 15177520
(43) Date of publication of application: 16.05.2018
(73) Proprietor: Kuros Biosciences AG, 8952 Schlieren (CH)
(72) Inventor: HENNECKE, Frank, 8305 Dietlikon (CH); KINZLER, Matthias, 8805 Richterswil (CH); SAUDAN, Philippe, 8422 Pfungen (CH); ERICKSON, Jennifer, Rhode Island, RI 02911 (US); LACAN, Isabelle, Norwood, MA 02062 (US); LAN LE, Chi, Andover, MA 01810 (US)
(74) Representative: Latscha Schöllhorn Partner AG
(86) International application number: PCT/EP2016/066044
(87) International publication number: WO 2017/005818

(56) References cited:
- EP-A1- 1 717 243
- WO-A2-2008/073960

### SEQUENCE LISTING

<110> Kuros Biosciences AG
<120> GUANINE-RICH OLIGONUCLEOTIDES
<130> P3339PC00
<150> US 62/189,832
   <151> 2015-07-08
<150> EP15177520
   <151> 2015-07-20
<160> 11
<170> PatentIn version 3.5
<210> 1
   <211> 30
   <212> DNA
   <213> artificial sequence
<220>
   <223> G-rich oligonucleotide G10
<400> 1
   gggggggggg gacgatcgtc gggggggggg 30
<210> 2
   <211> 10
   <212> DNA
   <213> artificial sequence
<220>
   <223> palindromic sequence
<400> 2
   gacgatcgtc 10
<210> 3
   <211> 19
   <212> DNA
   <213> artificial sequence
<220>
   <223> G-rich oligonucleotide
<400> 3
   ggggacgatc gtcgggggg 19
<210> 4
   <211> 20
   <212> DNA
   <213> artificial sequence
<220>
   <223> G-rich oligonucleotide
<400> 4
   gggggacgat cgtcgggggg 20
<210> 5
   <211> 21
   <212> DNA
   <213> artificial sequence
<220>
   <223> G-rich oligonucleotide
<400> 5
   ggggggacga tcgtcggggg g 21
<210> 6
   <211> 22
   <212> DNA
   <213> artificial sequence
<220>
   <223> G-rich oligonucleotide
<400> 6
   gggggggacg atcgtcgggg gg 22
<210> 7
   <211> 24
   <212> DNA
   <213> artificial sequence
<220>
   <223> G-rich oligonucleotide
<400> 7
   ggggggggac gatcgtcggg gggg 24
<210> 8
   <211> 26
   <212> DNA
   <213> artificial sequence
<220>
   <223> G-rich oligonucleotide
<400> 8
   ggggggggga cgatcgtcgg gggggg 26
<210> 9
   <211> 28
   <212> DNA
   <213> artificial sequence
<220>
   <223> G-rich oligonucleotide
<400> 9
   gggggggggg acgatcgtcg gggggggg 28
<210> 10
   <211> 30
   <212> DNA
   <213> artificial sequence
<220>
   <223> G-rich oligonucleotide
<400> 10
   ggggggcgac gacgatcgtc gtcggggggg 30
<210> 11
   <211> 20
   <212> DNA
   <213> artificial sequence
<220>
   <223> Guanine-rich oligonucleotides
<400> 11
   ggtgcatcga tgcagggggg 20

## Description

### FIELD OF THE INVENTION

This invention relates to methods for oligonucleotide synthesis, specifically the synthesis of oligonucleotides that contain a high content of guanine monomers. In more detail, the invention relates to a method for coupling a nucleoside phosphoramidite during the synthesis of an oligonucleotide to a universal support, to a first nucleoside, or to an extending oligonucleotide.

### BACKGROUND OF THE INVENTION

Chemically synthesized DNAs and RNAs ("oligonucleotides") and analogs thereof are used in most molecular biological applications. Methods of oligonucleotide synthesis have been available for over thirty years (see Agarwal et al., Nature, 227:27-34 (1970)), and still the most common method of oligonucleotide synthesis is through phosphoramidite chemistry (see McBride et al., Tetrahedron Lett., 24:245-248 (1983); Beaucage et al., Curr Protoc Nucleic Acid Chem 3.3.1-3.3.20 (2000); US 5,750,666.

Phosphoramidite synthesis typically begins with the 3'-most nucleotide and proceeds through a series of cycles composed of four steps that are repeated until the 5'-most nucleotide is attached. However, it is within the ordinary skill of the artisan to establish phosphoramidite synthesis in 5'-3' direction by choosing the first nucleoside and the nucleoside phosphoramidite in the appropriate conformation. The methods disclosed herein are applicable in both directions of synthesis, wherein synthesis in 3'-5' direction is generally preferred.

The four steps are deprotection, coupling, capping and stabilization (generally oxidation or sulfurization). In one variation, during the deprotection step the trityl group attached to the 5'-carbon of the pentose sugar of the recipient nucleotide is removed by trichloroacetic acid (TCA) or dichloroacetic acid (DCA) in a suitable solvent such as dichloromethane or toluene, leaving a reactive hydroxyl group. The next phosphoramidite monomer is added in the coupling step. An activator such as tetrazole, a weak acid, is used to react with the coupling nucleoside phosphoramidite, forming a tetrazolyl phosphoramidite intermediate. This intermediate then reacts with the hydroxyl group of the recipient and the 5' to 3' linkage is formed. The tetrazole is reconstituted and the process continues. A coupling failure results in an oligonucleotide still having a reactive hydroxyl group on the 5'-end. To prevent these oligonucleotides from remaining reactive for the next cycle (which would produce an oligonucleotide with a missing nucleotide), they are removed from further synthesis by being irreversibly capped by an acetylating reagent such as a mixture of acetic anhydride and N-methylimidazole. This reagent reacts only with the free hydroxyl groups to cap the oligonucleotides. In the oxidation step, the phosphite linkage between the growing oligonucleotide and the most recently added nucleotide is stabilized, typically in the presence of iodine as a mild oxidant in tetrahydrofuran (THF) and water. The water acts as the oxygen donor and the iodine forms an adduct with the phosphorous linkage. The adduct is decomposed by the water leaving a stable phosphotriester linkage.

There have been many significant modifications to phosphoramidite synthesis in order to reduce synthesis time and create a higher yield of product. Modified phosphoramidite monomers have been developed that also require additional modifications to synthesis.

However, some problems still remain in the synthesis of certain oligonucleotides. One issue has been the synthesis of guanine (G)-rich oligonucleotides. Oligonucleotides with G-rich regions have been very promising for a variety of applications. G-rich oligonucleotides generally fold into complex structures that have useful applications in molecular biology and medicine. A variety of aptamers have been selected that fold into tightly-packed 4-stranded structures (e.g. thrombin aptamer). The G-rich repeats in nucleic acids form these tetraplexes in the presence of certain monovalent or divalent metal ions with a variety of biological roles (see Deng et al., PNAS (2001), 98, 13665-13670; Jin et al., PNAS (1992), 89, 8832-8836; and Lee, Nucleic Acids Research (1990), 18, 6057-6060.

High quality synthesis for guanine (G)-rich oligonucleotides, in particular with consecutive guanine residues, is difficult to achieve, likely due to the poor accessibility of the 5'-hydroxyl group by the activated phosphoramidite in the coupling step. In particular, the support-bound protected G-rich oligomer undergoes some aggregation or has solubility problems in acetonitrile after a certain length or base composition is reached, which is the likely cause of poor accessibility of the 5'-hydroxyl group. This leads to impurities and synthesis failures such as oligonucleotides missing one or more nucleotides such as one or more ending guanine (G)-residues or to oligonucleotides having one or more nucleotides such as one or more guanine (G)-residues in addition. These impurities and synthesis failures, however, further leads to a decrease in the desired full length product (FLP) due to the challenging purification and separation of those impurities and synthesis failures from the FLP.

The standard solvent for oligonucleotide synthesis is acetonitrile. However, WO 2008/073960 proposed methods for the oligonucleotide synthesis, in particular for the synthesis of G-rich oligonucleotides using phosphoramidite chemistry, and suggests the use of alternative solvents such as polar aprotic solvents. In particular, the use of sulfolane during the coupling step has been suggested to alleviate aggregation or solubility issues with oligomers rich in guanine monomers. Typically, sulfolane has been used in a 1:1 solvent mixture with acetonitrile to provide better solubility for oligomers, in particular for oligomers with a high content of guanine residues.

Even though, WO 2008/073960 was able to reduce the amount of impurities and synthesis failures and to improve the crude quality of the synthesized G-rich oligonucleotides, there is still a need to further improve the synthesis of this important class of oligonucleotides, in particular, in terms of reducing impurities and synthesis failures as well as in terms of purity and yield of the desired oligonucleotide products.

### SUMMARY OF THE INVENTION

We have now surprisingly found that the use of *N*,*N-*dimethylformamide (DMF), preferably in a solvent mixture with acetonitrile or even further preferably as the sole solvent, for the synthesis of G-rich oligonucleotides using phosphoramidite chemistry not only reduces impurities and synthesis failures but, furthermore, leads to a higher purity and yield of the synthesized oligonucleotide. In particular, the better quality crude oligonucleotide obtained with the methods of the present invention facilitates the purification of the crude and leads, thus, to a higher purity and yield of the synthesized oligonucleotide. Not only, thus, enable the methods of the present invention higher scale production but the inventive methods are especially important when the oligonucleotides are intended for pharmaceutical use, including therapeutic use.

Therefore, in a first aspect, the present invention provides for a method for coupling a nucleoside phosphoramidite during the synthesis of an oligonucleotide to a universal support, to a first nucleoside, or to an extending oligonucleotide, wherein said oligonucleotide comprises a region of 3 or more consecutive guanine monomers, and wherein said method comprising the steps of (i) generating a coupling solution, wherein said coupling solution comprises: (a) said nucleoside phosphoramidite; (b) an activating reagent; and (c) one or more solvents, wherein one of said one or more solvents is *N*,*N-*dimethylformamide (DMF), and wherein preferably the volume of said DMF is equal to or higher than 25%, further preferably equal to or higher than 33%, and again further prefereably equal to or higher than 50%, of the total volume of said one or more solvents; and (ii) contacting said coupling solution with said universal support, with said first nucleoside, or with said extending oligonucleotide.

In particular, the use of *N*,*N-*dimethylformamide (DMF), preferably in a solvent mixture with acetonitrile or even further preferably as the sole solvent, for coupling a nucleoside phosphoramidite during the synthesis of an oligonucleotide to a universal support, to a first nucleoside, or to an extending oligonucleotide, has been found to be highly beneficial.

Without being bound by this theory, it is believed that DMF is expected to avoid formation of tetraplex structures typically formed by G-rich oligonucleotides, and therefore facilitate the coupling of the next amidite during chain elongation. Besides alleviating aggregation, the inventive methods are believed to provide better solubility for oligonucleotides rich in guanine monomers.

In a second aspect, the present invention provides for a method for producing an oligonucleotide, said method comprising any one of the methods described herein for coupling a nucleoside phosphoramidite during the synthesis of an oligonucleotide to a universal support, to a first nucleoside, or to an extending oligonucleotide in accordance with said first aspect of the present invention.

In a third aspect, the present invention provides for method for producing an oligonucleotide, said method comprising (i) coupling a nucleoside phosphoramidite to a universal support or to a first nucleoside; wherein said coupling comprises any one of the methods described herein for coupling a nucleoside phosphoramidite during the synthesis of an oligonucleotide to a first nucleoside in accordance with said first aspect of the present invention; (ii) generating an extending oligonucleotide by oxidizing the product of step (i); (iii) coupling a nucleoside phosphoramidite to the product of step (ii) after deprotection; wherein said coupling comprises the method of said first aspect of the present invention; (iv) generating an extending oligonucleotide by oxidizing the product of step (iii); and (v) repeating steps (iii) and (iv) until said extending oligonucleotide comprises the sequence of said oligonucleotide.

Further aspects of the present invention and preferred embodiments thereof will become apparent as this specification proceeds.

### DETAILED DESCRIPTION OF THE INVENTION

Unless defined otherwise, all technical and scientific terms used herein have the same meanings as commonly understood by one of ordinary skill in the art to which this invention belongs.

"Oligonucleotide". As used herein, the term "oligonucleotide" refers to a nucleic acid sequence comprising 2 or more nucleotides, preferably 6 to 200 nucleotides, further preferably 10 to 100, and again more preferably 20 to about 100 nucleotides, again more preferably 20 to 50, and again further preferably 20 to 40 nucleotides. Very preferably, oligonucleotides comprise about 30 nucleotides, more preferably oligonucleotides comprise exactly 30 nucleotides, and most preferably oligonucleotides consist of exactly 30 nucleotides. Further preferred oligonucleotides consist of 15, 16, 17, 18, 19, 20, 21, 22, 23, 24, 25, 26, 27, 28, 29, 30, 31, 32, 33, 34, 35, 36, 37, 38, 39, or 40 nucleotides. Oligonucleotides are polyribonucleotides or polydeoxribonucleotides and are preferably selected from (a) unmodified RNA or DNA, and (b) modified RNA or DNA. The modification may comprise the backbone or nucleotide analogues which are known to the person skilled in the art. Preferred nucleotide modifications/analogs are phosphorothioates or alkylphosphorothioates modifications. Besides unmodified oligonucleotides consisting exclusively of phosphodiester bound nucleotides, phosphothioated nucleotides are protected against degradation in a cell or an organism and are therefore preferred nucleotide modifications. Further encompassed are oligonucleotides comprising phosphodiester bound nucleotides and phosphothioated nucleotides. The term oligonucleotide as used herein typically and preferably refers to a single stranded deoxyribonucleotide. Typically, an oligonucleotide comprises a region of 3 or more consecutive guanine monomers. Preferably, an oligonucleotide comprises a first region of 3, 4, 5, 6, 7, 8, 9, 10, 11, 12, 13, 14, 15, 16, 17, 18, 19, or 20 consecutive guanine monomers, wherein preferred hereby said first region is located at the 3'-terminus of said oligonucleotide. In a further preferred embodiment said oligonucleotide comprises a second region of 3, 4, 5, 6, 7, 8, 9, 10, 11, 12, 13, 14, 15, 16, 17, 18, 19, or 20 consecutive guanine monomers, wherein preferably said second region is located at the 5'-terminus of said oligonucleotide. Preferably, an oligonucleotide comprises at least 30 % guanine monomers. A further preferred oligonucleotide comprises at least one poly G stretch as defined below. More preferred oligonucleotides comprise 2, 3, 4, 5, 6, 7, 8, 9, or 10 of said poly G stretches. Very preferred oligonucleotides comprise exactly two poly G stretches, wherein preferably one of said two poly G stretches is located at the 5' end or at the 3' end of said oligonucleotide. Even more preferred oligonucleotides comprise exactly two poly G stretches, wherein one of said two poly G stretches is located at the 5' end of said oligonucleotide and one of said two poly G stretches is located at the 3' end of said oligonucleotide. Very preferred oligonucleotides are unmethylated CpG containing oligonucleotides comprising at least one, preferably one, two, three or four CpG motifs. Still more preferred oligonucleotides comprise a palindromic sequence, wherein preferably said palindromic sequence comprises least one, preferably one, two, three or four CpG motifs. Still more preferred oligonucleotides comprise a palindromic sequence, wherein preferably said palindromic sequence comprises, or preferably consists of the sequence GACGATCGTC (SEQ ID NO:2). Still more preferred oligonucleotides comprise a palindromic sequence, wherein said palindromic sequence is flanked at its 5' end by a poly G stretch and wherein said palindromic sequence is flanked at its 3' end by a poly G stretch, wherein preferably said palindromic sequence is GACGATCGTC (SEQ ID NO:2). Very preferred oligonucleotides comprise a palindromic sequence, wherein said palindromic sequence is flanked at its 5' end by at least 3 to 10, preferably by 4 to 10 guanosine entities and wherein said palindromic sequence is flanked at its 3' end at least 3 to 10, preferably by 4 to 10, guanosine entities, wherein preferably said palindromic sequence is GACGATCGTC (SEQ ID NO:2).

"Poly G stretch": The term poly G stretch, as used herein, refers to a segment of an oligonucleotide, wherein said segment consists of at least 3 consecutive guanosine residues. Preferred poly G stretches consist of 3 to 25, preferably of 4 to 20, more preferably of 4 to 15 and most preferably of 4 to 10 consecutive guanosine entities. Further preferred poly G stretches consist of 3, 4, 5, 6, 7, 8, 9, 10, 11, 12, 13, 14, 15, 16, 17, 18, 19, or 20 consecutive guanosine entities.

"CpG motif': As used herein, the term "CpG motif' refers to an oligodesoxynucleotide containing at least one unmethylated cytosine, guanine dinucleotide and wherein preferably said CG dinucleotide is phosphodiester bound.

"Unmethylated CpG-containing oligonucleotide": As used herein, the term "unmethylated CpG-containing oligonucleotide" or "CpG" refers to an oligonucleotide, preferably to an oligodesoxynucleotide, containing at least one CpG motif. Preferably, CpG relates to a single stranded oligodesoxynucleotide containing an unmethylated cytosine followed 3' by a guanosine, wherein said unmethylated cytosine and said guanosine are linked by a phosphate bond, wherein preferably said phosphate bound is a phosphodiester bound or a phosphothioate bound, and wherein further preferably said phosphate bond is a phosphodiester bound.

In a first aspect, the present invention provides for a method for coupling a nucleoside phosphoramidite during the synthesis of an oligonucleotide to a universal support, to a first nucleoside, or to an extending oligonucleotide, wherein said oligonucleotide comprises a region of 3 or more consecutive guanine monomers, and wherein said method comprising the steps of (i) generating a coupling solution, wherein said coupling solution comprises: (a) said nucleoside phosphoramidite; (b) an activating reagent; and (c) one or more solvents, wherein one of said one or more solvents is *N*,*N-*dimethylformamide (DMF); and (ii) contacting said coupling solution with said universal support, with said first nucleoside, or with said extending oligonucleotide.

In a preferred embodiment, the volume of said DMF is equal to or higher than 25%, preferably equal to or higher than 33% of the total volume of said one or more solvents. In another preferred embodiment, the volume of said DMF is equal to or higher than 50% of the total volume of said one or more solvents.

In another preferred embodiment, said one or more solvents further comprises aectontirile, wherein the volume of said acetonitrile is lower than or at most equal to 75%, preferably lower than or at most equal to 67%, and further preferably lower than or at most equal to 50%, of the total volume of said one or more solvents.

In another preferred embodiment, said one or more solvents comprises, preferably consists of, DMF and acetonitrile, and wherein the ratio (v/v) of said DMF to acetonitrile is between 1:3 and 3:1. In another preferred embodiment, said one or more solvents consists of DMF and acetonitrile, and wherein the ratio (v/v) of said DMF to acetonitrile is 1:1.

In another preferred embodiment, the volume of said DMF is equal to or higher than 67%, preferably equal to or higher than 75%, further prefereably equal to or higher than 90%, of the total volume of said one or more solvents.

In a very preferred embodiment, said one or more solvents consists of exactly one solvent, wherein said exactly one solvent is DMF. Thus, in a very preferred embodiment, said coupling solution comprises exactly one solvent, and wherein said exactly one solvent is DMF. Preferably, said DMF has a purity of at least 98%, preferably of at least 99%, again more preferably of at least 99.5%, and again more preferably of at least 99.8%.

Suitable activators are known to the person skilled in the art and are described, by way of example, in US 6,031,092 and US 6,476,216, each of which is incorporated herein by reference. In a preferred embodiment, said activating reagent is selected from (a) 4,5-dicyanoimidazole (DCI); (b) 5-ethylthio-1*H*-tetrazole (ETT); (c) 5-benzylthio-1*H*-tetrazole (BTT); or (d) 5-(3,5-*bis*-trifluoromethyl)phenyl-1*H-*tetrazole (Activator 42). In another preferred embodiment, said activating reagent is selected from (a) 5-ethylthio-1*H*-tetrazole (ETT); (b) 5-benzylthio-1*H-*tetrazole (BTT); or (c) 5-(3,5-bis-trifluoromethyl)phenyl-1*H*-tetrazole (Activator 42), and wherein preferably said activating reagent is 5-ethylthio-1*H*-tetrazole (ETT). In another preferred embodiment, said activating reagent is 4,5-dicyanoimidazole (DCI) or 5-ethylthio-1*H*-tetrazole (ETT). In a very preferred embodiment said activating reagent is 5-ethylthio-1*H*-tetrazole (ETT). In again a very preferred embodiment, said coupling solution comprises, preferably consists of, (a) said nucleoside phosphoramidite; (b) said activating reagent, wherein said activating reagent is is 5-ethylthio-1*H*-tetrazole (ETT); and (c) exactly one solvent, and wherein said exactly one solvent is DMF. In another preferred embodiment, the concentration of said activating reagent in said coupling solution is 0.05 to 0.90 M, and wherein preferably the concentration of said activating reagent in said coupling solution is 0.40 to 0.80 M.

In a further preferred embodiment, the concentration of said nucleoside phosphoramidite in said coupling solution is at least 0.03 M, and wherein preferably the concentration of said nucleoside phosphoramidite in said coupling solution is 0.03 to 0.60 M, and wherein further preferably the concentration of said nucleoside phosphoramidite in said coupling solution is 0.03 to 0.30 M.

In a further preferred embodiment, said first nucleoside and/or said extending oligonucleotide is immobilized on a support, wherein preferably said support is selected from (a) polymeric support, preferably polystyrene support; and (b) silica support, preferably a controlled pore glass (CPG) support. In a further preferred embodiment, said first nucleoside and/or said extending oligonucleotide is immobilized on a support, wherein preferably said support is selected from (a) polystyrene support; and (b) silica support, preferably a controlled pore glass (CPG) support. Some polymeric bead supports are disclosed in the following patents: US 6,016,895; US 6,043,353; US 6,300,486; US 8,541,599; and US 8,153,725 B2 .

In a very preferred embodiment, said support is a polystyrene support. In a further very preferred embodiment, said support is a polystyrene support, wherein said polystyrene support is cross-linked by divinylbenzene, wherein preferably said polystyrene support is characterized by functional hydroxyl groups; and wherein further preferably said polystyrene support comprises an average particle size of about 80-90 µm. Said supports are known to the person skilled in the art. One of these preferred supports for the present invention are NittoPhase®HL Solid Supports by Nitto Denko Corporation. These have been used for the examples provided in the present invention.

Such supports are generally used in the art and typically and preferably further comprises a linker, typically and preferably a succinate-linker or a linker comprising a succinate moiety. Thus, in a preferred embodiment of the present invention, said support further comprises a linker, wherein preferably said linker comprises a succinate moiety.

In a very preferred embodiment, the support further comprises a linker, wherein the linker is represented by the following formula I 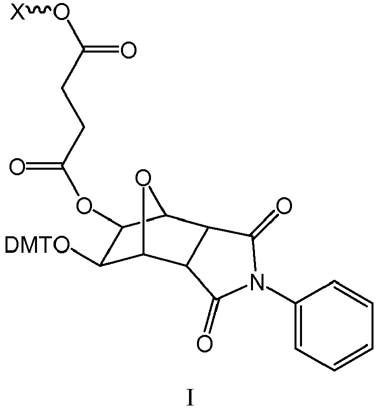 and wherein X represents said support, wherein preferably X represents said polystyrene support cross-linked by divinylbenzene.

In a very preferred embodiment, said support further comprises a linker, wherein said support is a polystyrene support, wherein said polystyrene support is cross-linked by divinylbenzene, and wherein said linker is represented by the formula I, wherein X represents said polystyrene support cross-linked by divinylbenzene. Said very preferred support with said linker allows a preferred loading capacity of 200-400 µmol/g. Further very preferred are said support with said linker as used in the example section (NPHL250), wherein said support with linker allows a loading capacity of 250 µmol/g. These very preferred support-linker combinations are commercially available from Kinovate Life Sciences, Inc., Oceanside, CA 92058 and named UnyLinker™ loaded NittoPhase®HL & NittoPhase® Solid Supports.

Various linkers for oligomer solid phase synthesis have been described and are known by the person skilled in the art. Preferred linkers and support-linker combinations for the present invention are disclosed in WO 2005/049621. Furthermore, the synthesis of these linkers and support/linker combinations are also described in WO 2005/049621.

The synthesis primers known by the person skilled in the art may typically comprise besides the support-linker combinations a suitable nucleoside depending on the sequence of the oligonucleotide to be actually synthesized. These synthesis primers are typically prepared by covalently linking said suitable nucleoside to said support through said linker. Numerous of said synthesis primers can even be commercially purchased.

In a preferred embodiment, said oligonucleotide comprises at least one poly G stretch. In another preferred embodiment, said oligonucleotide comprises at least 30 % guanine monomers. In a further preferred embodiment, said oligonucleotide comprises at least 40 % guanine monomers. In another preferred embodiment, said oligonucleotide comprises at least 50 % guanine monomers.

In a further preferred embodiment the oligonucleotide contains a first region of 3 or more consecutive guanine monomers. In a further preferred embodiment the oligonucleotide contains a first region of 4 or more consecutive guanine monomers. In a further preferred embodiment the oligonucleotide contains a first region of 5 or more consecutive guanine monomers. In another preferred embodiment, said oligonucleotide comprises a first region of 3, 4, 5, 6, 7, 8, 9, 10, 11, 12, 13, 14, 15, 16, 17, 18, 19, or 20 consecutive guanine monomers. Preferably, said first region is located at the 3'-terminus of said oligonucleotide.

In a further preferred embodiment the oligonucleotide contains a second region of 3 or more consecutive guanine monomers. In a further preferred embodiment the oligonucleotide contains a second region of 4 or more consecutive guanine monomers. In a further preferred embodiment the oligonucleotide contains a second region of 5 or more consecutive guanine monomers. In another preferred embodiment, said oligonucleotide comprises a second region of 3, 4, 5, 6, 7, 8, 9, 10, 11, 12, 13, 14, 15, 16, 17, 18, 19, or 20 consecutive guanine monomers. Preferably, said second region is located at the 5'-terminus of said oligonucleotide.

In another preferred embodiment, said oligonucleotide comprises a first region of 3 or more consecutive guanine monomers and a second region of 3 or more consecutive guanine monomers, and wherein said first region is located at the 3'-terminus of said oligonucleotide and wherein said second region is located at the 5'-terminus of said oligonucleotide.

In another preferred embodiment, said oligonucleotide comprises a first region of 4 or more consecutive guanine monomers and a second region of 4 or more consecutive guanine monomers, and wherein said first region is located at the 3'-terminus of said oligonucleotide and wherein said second region is located at the 5'-terminus of said oligonucleotide.

In a further preferred embodiment said oligonucleotide comprises 10 to 50, preferably 20 to 40, and most preferably 30 nucleotide monomers.

In a further preferred embodiment said oligonucleotide comprises a palindromic sequence, wherein preferably said palindromic sequence is GACGATCGTC (SEQ ID NO:2). In another preferred embodiment, said palindromic sequence is flanked at its 5'-terminus by at least 4 and at most 20, preferably at most 10, guanosine entities. In another preferred embodiment, said palindromic sequence is flanked at its 3'-terminus by at least 4 and at most 20, preferably at most 10, guanosine entities.

In a further preferred embodiment said oligonucleotide comprises or preferably consists of a nucleotide sequence selected from the group consisting of: (a) GGGGACGATC GTCGGGGGG (SEQ ID NO:3); (b) GGGGGACGAT CGTCGGGGGG (SEQ ID NO:4); (c) GGGGGGACGATCGTCGGGGGG (SEQ ID NO:5); (d) GGGGGGGACG ATCGTCGGGG GG (SEQ ID NO:6); (e) GGGGGGGGAC GATCGTCGGG GGGG (SEQ ID NO:7); (f) GGGGGGGGGA CGATCGTCGG GGGGGG (SEQ ID NO:8); (g) GGGGGGGGGG ACGATCGTCG GGGGGGGG (SEQ ID NO:9); (h) GGGGGGGGGG GACGATCGTC GGGGGGGGGG (SEQ ID NO:1); and (i) GGGGGGCG ACGACGAT CGTCGTCG GGGGGG (SEQ ID NO: 10). In a very preferred embodiment, said oligonucleotide comprises or preferably consists of SEQ ID NO:1. In a further preferred embodiment the oligonucleotide is SEQ ID NO:10.

In a further preferred embodiment, said oligonucleotide is a deoxynucleotide, and wherein preferably said deoxynucleotide consists exclusively of phosphodiester bound monomers. More preferably, said oligonucleotide comprises or preferably consists of SEQ ID NO:1 and said oligonucleotide is a deoxynucleotide, and wherein said deoxynucleotide consists exclusively of phosphodiester bound monomers.

In a further aspect, the invention relates to a method for producing an oligonucleotide, said method comprising any one of the methods described herein for coupling a nucleoside phosphoramidite during the synthesis of an oligonucleotide to an universal support, to a first nucleoside, or to an extending oligonucleotide.

In a further aspect, the invention relates to a method for producing an oligonucleotide, said method comprising (i) coupling a nucleoside phosphoramidite to a first nucleoside; wherein said coupling comprises any one of the methods described herein for coupling a nucleoside phosphoramidite during the synthesis of an oligonucleotide to a first nucleoside; (ii) generating an extending oligonucleotide by oxidizing the product of step (i); (iii) coupling a nucleoside phosphoramidite to the product of step (ii), typically and preferably after deprotection; wherein said coupling comprises any one of the methods described herein for coupling a nucleoside phosphoramidite during the synthesis of an oligonucleotide to an extending oligonucleotide; (iv) generating an extending oligonucleotide by oxidizing the product of step (iii); and (v) repeating steps (iii) and (iv) until said extending oligonucleotide comprises the sequence of said oligonucleotide.

In a preferred embodiment said method further comprises the step of purifying said oligonucleotide under denaturing conditions, wherein preferably said denaturing conditions are characterized by a pH of 10 to 14, preferably by a pH of 10 to 13, more preferably by a pH of about 12, most preferably by a pH of 12.

In a further preferred embodiment said method further comprises the step of purifying said oligonucleotide at a pH of 10 to 14, preferably at a pH of 10 to 13, more preferably at a pH of about 12, most preferably at a pH of 12.

In a further preferred embodiment, said purification is performed by anion-exchange chromatography, wherein preferably said anion-exchange chromatography is performed using an anion-exchange matrix functionalized with quaternary amine groups, wherein further preferably said anion-exchange matrix is composed of a material selected from polystyrene, polystyrene/divinyl benzene or polymethacrylate, and wherein still further preferably said material is polystyrene/divinyl benzene.

In a further preferred embodiment said oligonucleotide is produced in a molar yield with respect to said first nucleoside of at least 15%, preferably of at least 20 %, again preferably of at least 25 %. Still more preferably of at least 30%.

In a further preferred embodiment the purity of said oligonucleotide is at least 75 %, preferably at least 80 %, more preferably at least 85 %, still more preferably at least 90 %, and most preferably at least 95 %.

The inventive methods disclosed herein are well suited for the large scale synthesis of oligonucleotides, in particular of G-rich oligonucleotides, such as, for example, poly-G flanked unmethylated CpG containing oligonucleotides. Such compounds are, in particular, used in pharmaceutical applications.

The following examples further illustrate the invention but, of course, should not be construed as in any way limiting its scope.

### EXAMPLES

The following examples further illustrate the invention but should not be construed as in any way limiting its scope.

The following abbreviations have particularly been used throughout this Example section and the entire specification:
- ACN: Acetonitrile
- CNET: Cyanoethyl protecting group
- CV: Column volume(s)
- DCA: Dichloroacetic acid
- DCI: Dicyanoimidazole
- DEA: Diethylamine
- DMF: Dimethylformamide
- eq: equivalents
- ETT: 3-Ethylthio-1H-tetrazole
- FLP: Full length product
- HPLC: High pressure liquid chromatography
- NMI: N-methylimidazole
- NPHL 250: Nittophase high loaded unylinker 250
- OD: Optical density
- TBA: Tert-Butylamine
- UV: Ultraviolet

Dimethylformamide (DMF) was purchased from Acros Organics (part of Thermo Fisher Scientific) and had a purity of 99.8%. Nittophase high loaded unylinker 250 (NPHL 250) was purchased from Kinovate Life Sciences, Inc., Oceanside, CA.

### EXAMPLE 1

This example describes the synthesis of oligonucleotide G10 (SEQ ID NO:1) in the presence of various solvents and co-solvent combinations. It demonstrates the superiority of the solvent mixture ACN/DMF and pure DMF over ACN/sulfolane in terms of purity of full-length product. It further demonstrates the superiority of pure DMF over ACN/DMF in terms of total oligonucleotide yield.

### Synthesis in ACN/sulfolane with DCI as activator

This example describes a 1.51 mmol trityl-off synthesis of oligonucleotide G10 (SEQ ID NO:1) on an Äkta 100 OligoPilot using Nittophase High Loaded Unylinker 250 (NPHL 250) as solid synthesis support. 57.7 ml of the synthesis support, swollen in ACN/sulfolane (1:1 v/v) (support density: 0.109 g/ml) were filled into the synthesis column (column diameter 3.5 cm, column height 6 cm), and after a pre-synthesis wash with ACN (64.1 ml/min, 2 CV) the following synthesis cycle was used: (1) detritylation with DCA in toluene (first cycle: 10% DCA in toluene, 32.9 ml/min; subsequent cycles: 3% DCA in toluene, 64.1 ml/min; 2 CV) followed by ACN wash (2 CV at 32.1 ml/min and 2 CV at 64.1 ml/min); (2) conditioning with ACN/sulfolane (1:1 v/v; 32.9 ml/min, 1.5 CV); (3) coupling (activator: 0.7 M DCI in ACN/sulfolane (1:1 v/v); deoxynucleoside phosphoramidite (2.0 eq/support): 0.2 M in ACN/sulfolane (1:1 v/v); charge volume: 30.2 ml (15.1 ml amidite + 15.1 ml activator), charge flow rate: 53.1 ml/min; push volume: 8 ml, push flow rate: 53.1 ml/min; recycle time: 10 min, recycle flow rate: 48.1 ml/min) followed by ACN wash (1 CV, 64.1 ml/min); (4) pre-oxidation capping with 0.2 CV Cap A (NMI/pyridine/ACN, 2:3:5 v/v/v)/ACN (1:1 v/v) followed by 0.75 CV of 1:1 (v/v) Cap A/Cap B (Isobutyric anhydride/ACN 1:1 v/v), contact time 2.5 min, charge/push flow rate: 17.3 ml/min, push volume: 1.1 ml, wash volume: 1 CV, wash flow rate: 64.1 ml/min; (5) pre-oxidation push with 0.5 CV Cap A (34.8 ml/min); (6) oxidation with 50 mM 12 (3 eq) in pyridine/water (9:1 v/v), charge volume: 90.6 ml, contact time: 2.6 min, charge/push flow rate: 34.8 ml/min, oxidation push volume: 1.1 CV, followed by ACN wash (1 CV, 64.1 ml/min); (7) pre-capping conditioning with 1.5 CV ACN/sulfolane (1:1 v/v, 32.9 ml/min); (8) capping with 0.2 CV Cap A/ACN (1:1 v/v) followed by 0.75 CV of 1:1 (v/v) Cap A/Cap B, contact time 2.5 min, charge/push flow rate: 17.3 ml/min, push volume: 1.1 CV, wash volume: 1 CV, wash flow rate: 64.1 ml/min. After a final column wash with 2 CV ACN (64.1 ml/min) and the final detritylation, the CNET protecting group was removed from the phosphodiester linkage with 20% DEA in ACN (5 CV, contact time 10 min, followed by a wash with 4 CV ACN (96.2 ml/min)). Cleavage and deprotection was achieved by treatment with not less than 4 CV of 28-30% ammonia at 50°C for not less than 24 hours in a bottle on a shaker table followed by repeated washing steps of 1 CV water each until a UV reading of less than 40 OD/ml is reached.

The OD was determined by absorption measurement at 260 nm. The oligonucleotide yield (OD/µmol) is given in Table 1.

Anion exchange analysis for determination of the crude oligonucleotide purity was performed on a Waters Alliance HPLC system on a Dionex DNAPac PA200 4x250 mm column at 30°C. The samples are diluted to 6.25 OD/ml in HPLC buffer A (20 mM NaOH), 20 µl of which were injected onto the column and separated at a flow rate of 1 ml/min using a gradient from 25 to 40% HPLC buffer B (20 mM NaOH, 1.5 M NaCl, 40% Methanol) during 5 min followed by a gradient from 40 to 55% buffer B during 35 min. The % full length product is given in Table 1.

### Synthesis in ACN/DMF with DCI as activator

This example describes a 1.51 mmol trityl-off synthesis of SEQ ID NO:1 on an Äkta 100 OligoPilot using Nittophase High Loaded Unylinker 250 (NPHL 250) as solid synthesis support. 57.7 ml of the synthesis support, swollen in ACN/DMF (1:1 v/v) (support density: 0.109 g/ml) were filled into the synthesis column (column diameter 3.5 cm, column height 6 cm), and after a pre-synthesis wash with ACN (64.1 ml/min, 2 CV) the following synthesis cycle was used: (1) detritylation with DCA in toluene (first cycle: 10% DCA in toluene, 32.9 ml/min; subsequent cycles: 3% DCA in toluene, 64.1 ml/min; 2 CV) followed by ACN wash (2 CV at 32.1 ml/min and 2 CV at 64.1 ml/min); (2) conditioning with ACN/DMF (1:1 v/v; 32.9 ml/min, 1.5 CV); (3) coupling (activator: 0.7 M DCI in ACN/DMF (1:1 v/v); deoxynucleoside phosphoramidite (2.0 eq/support): 0.2 M in ACN/DMF (1:1 v/v); charge volume: 30.2 ml (15.1 ml amidite + 15.1 ml activator), charge flow rate: 53.1 ml/min; push volume: 8 ml, push flow rate: 53.1 ml/min; recycle time: 10 min, recycle flow rate: 48.1 ml/min) followed by ACN wash (1 CV, 64.1 ml/min); (4) pre-oxidation capping with 0.2 CV Cap A (NMI/pyridine/ACN, 2:3:5 v/v/v)/ACN (1:1 v/v) followed by 0.75 CV of 1:1 (v/v) Cap A/Cap B (Isobutyric anhydride/ACN 1:1 v/v), contact time 2.5 min, charge/push flow rate: 17.3 ml/min, push volume: 1.1 ml, wash volume: 1 CV, wash flow rate: 64.1 ml/min; (5) pre-oxidation push with 0.5 CV Cap A (34.8 ml/min); (6) oxidation with 50 mM 12 (3 eq) in pyridine/water (9:1 v/v), charge volume: 90.6 ml, contact time: 2.6 min, charge/push flow rate: 34.8 ml/min, oxidation push volume: 1.1 CV, followed by ACN wash (1 CV, 64.1 ml/min); (7) pre-capping conditioning with 1.5 CV ACN/DMF (1:1 v/v, 32.9 ml/min); (8) capping with 0.2 CV Cap A/ACN (1:1 v/v) followed by 0.75 CV of 1:1 (v/v) Cap A/Cap B, contact time 2.5 min, charge/push flow rate: 17.3 ml/min, push volume: 1.1 CV, wash volume: 1 CV, wash flow rate: 64.1 ml/min. After a final column wash with 2 CV ACN (64.1 ml/min) and the final detritylation, the CNET protecting group was removed from the phosphodiester linkage with 20% DEA in ACN (5 CV, contact time not less than 45 min, followed by a wash with 4 CV ACN (96.2 ml/min)). Cleavage and deprotection was achieved by on-column recirculation with not less than 4 CV of 28-30% ammonia at 50°C followed by repeated washing steps of 1 CV water each until a UV reading of less than 40 OD/ml is reached.

The OD was determined by absorption measurement at 260 nm. The oligonucleotide yield (OD/µmol) is given in Table 1.

Anion exchange analysis for determination of the crude oligonucleotide purity was performed on a Waters Alliance HPLC system on a Dionex DNAPac PA200 4x250 mm column at 30°C. The samples are diluted to 6.25 OD/ml in HPLC buffer A (20 mM NaOH), 20 µl of which were injected onto the column and separated at a flow rate of 1 ml/min using a gradient from 25 to 40% HPLC buffer B (20 mM NaOH, 1.5 M NaCl, 40% Methanol) during 5 min followed by a gradient from 40 to 55% buffer B during 35 min. The % full length product is given in Table 1.

### Synthesis in DMF with DCI as activator

This example describes a 1.51 mmol trityl-off synthesis of SEQ ID NO:1 on an Äkta 100 OligoPilot using Nittophase High Loaded Unylinker 250 (NPHL 250) as solid synthesis support. 57.7 ml of the synthesis support, swollen in DMF (support density: 0.109 g/ml) were filled into the synthesis column (column diameter 3.5 cm, column height 6 cm), and after a pre-synthesis wash with ACN (64.1 ml/min, 2 CV) the following synthesis cycle was used: (1) detritylation with DCA in toluene (first cycle: 10% DCA in toluene, 32.9 ml/min; subsequent cycles: 3% DCA in toluene, 64.1 ml/min; 2 CV) followed by ACN wash (2 CV at 32.1 ml/min and 2 CV at 64.1 ml/min); (2) conditioning with DMF (32.9 ml/min, 1.5 CV); (3) coupling (activator: 0.7 M DCI in DMF; deoxynucleoside phosphoramidite (2.0 eq/support): 0.2 M in DMF; charge volume: 30.2 ml (15.1 ml amidite + 15.1 ml activator), charge flow rate: 53.1 ml/min; push volume: 8 ml, push flow rate: 53.1 ml/min; recycle time: 10 min, recycle flow rate: 48.1 ml/min) followed by ACN wash (1 CV, 64.1 ml/min); (4) pre-oxidation capping with 0.2 CV Cap A (NMI/pyridine/ACN, 2:3:5 v/v/v)/ACN (1:1 v/v) followed by 0.75 CV of 1:1 (v/v) Cap A/Cap B (Isobutyric anhydride/ACN 1:1 v/v), contact time 2.5 min, charge/push flow rate: 17.3 ml/min, push volume: 1.1 ml, wash volume: 1 CV, wash flow rate: 64.1 ml/min; (5) pre-oxidation push with 0.5 CV Cap A (34.8 ml/min); (6) oxidation with 50 mM 12 (3 eq) in pyridine/water (9:1 v/v), charge volume: 90.6 ml, contact time: 2.6 min, charge/push flow rate: 34.8 ml/min, oxidation push volume: 1.1 CV, followed by ACN wash (1 CV, 64.1 ml/min); (7) pre-capping conditioning with 1.5 CV DMF (32.9 ml/min); (8) capping with 0.2 CV Cap A/ACN (1:1 v/v) followed by 0.75 CV of 1:1 (v/v) Cap A/Cap B, contact time 2.5 min, charge/push flow rate: 17.3 ml/min, push volume: 1.1 CV, wash volume: 1 CV, wash flow rate: 64.1 ml/min. After a final column wash with 2 CV ACN (64.1 ml/min) and the final detritylation, the CNET protecting group was removed from the phosphodiester linkage with 20% TBA in ACN (5 CV, contact time not less than 45 min, followed by a wash with 4 CV ACN (96.2 ml/min)). Cleavage and deprotection was achieved by on-column recirculation with not less than 4 CV of 28-30% ammonia at 50°C followed by repeated washing steps of 1 CV water each until a UV reading of less than 40 OD/ml is reached.

The OD was determined by absorption measurement at 260 nm. The oligonucleotide yield (OD/µmol) is given in Table 1.

Anion exchange analysis for determination of the crude oligonucleotide purity was performed on a Waters Alliance HPLC system on a Dionex DNAPac PA200 4x250 mm column at 30°C. The samples are diluted to 6.25 OD/ml in HPLC buffer A (20 mM NaOH), 20 µl of which were injected onto the column and separated at a flow rate of 1 ml/min using a gradient from 25 to 40% HPLC buffer B (20 mM NaOH, 1.5 M NaCl, 40% Methanol) during 5 min followed by a gradient from 40 to 55% buffer B during 35 min. The % full length product is given in Table 1.

The data demonstrate that the purity of FLP in the crude synthesis is highest with DMF as co-solvent (57.9%) and with pure DMF as solvent (56.3%). Moreover, the total yield is highest with pure DMF as solvent (181 OD/µmol). Moreover, impurities and synthesis failures such as G10-1n and G10+1n, i.e. compounds comprising one or more G residues less or compounds comprising one or more G residues in addition to the targeted FLP, are strongly reduced when using DMF instead of ACN/sulfolane. The latter is in particular true for G10+1n which, in turn, is very beneficial due to the difficulty of separating the G10+1n compounds from the FLP.

**Table 1**

| **Solvent** | **Activator** | **Yield Oligo** [OD/µmol] | **Purity FLP** [% of oligo] | **Content FLP-1n relative to FLP** [%] | **Content FLP+1n relative to FLP** [%] |
|---|---|---|---|---|---|
| ACN/Sulfolane | DCI | 166 | 54.0 | 6.46 | 5.37 |
| ACN/DMF | DCI | 145 | 57.9 | 6.08 | 3.45 |
| DMF | DCI | 181 | 56.3 | 5.75 | 0.62 |

### EXAMPLE 2

This example describes the synthesis of SEQ ID NO:1 in the presence of pure DMF using ETT as activator. It demonstrates the superiority of ETT over DCI in terms of oligonucleotide yield and purity of full-length product.

### Synthesis in DMF with ETT as activator

This example describes a 1.26 mmol trityl-off synthesis of SEQ ID NO:1 on an Äkta 100 OligoPilot using Nittophase High Loaded Unylinker 250 (NPHL 250) as solid synthesis support. 48.1 ml of the synthesis support, swollen in DMF (support density: 0.109 g/ml) were filled into the synthesis column (column diameter 3.5 cm, column height 5 cm), and after a pre-synthesis wash with ACN (424 cm/hr, 2 CV) the following synthesis cycle was used: (1) detritylation with DCA in toluene (first cycle: 10% DCA in toluene, 50 cm/hr; subsequent cycles: 3% DCA in toluene, 424 cm/hr; 2 CV) followed by ACN wash (2 CV at 200 cm/hr and 2 CV at 424 cm/hr); (2) conditioning with DMF (205 cm/hr, 1.5 CV); (3) coupling (activator: 0.6 M ETT in DMF; deoxynucleoside phosphoramidite (2.0 eq/support): 0.2 M in DMF; charge volume: 26.4 ml (12.6 ml amidite + 13.8 ml activator), charge flow rate: 19.3 ml/min; push volume: 8 ml, push flow rate: 120 cm/hr; recycle time: 10 min, recycle flow rate: 212 cm/hr) followed by ACN wash (1 CV, 424 cm/hr); (4) pre-oxidation push with 0.5 CV Cap A (NMI/pyridine/ACN, 2:3:5 v/v/v, 29.0 ml/min); (5) oxidation with 50 mM 12 (3 eq) in pyridine/water (9:1 v/v), charge volume: 75.5 ml, contact time: 2.6 min, charge/push flow rate: 29.0 ml/min, oxidation push volume: 1.1 CV, followed by ACN wash (1 CV, 424 cm/hr); (6) pre-capping conditioning with 1.5 CV DMF (205 cm/hr); (7) capping with 0.2 CV Cap A/ACN (1:1 v/v) followed by 0.75 CV of 1:1 (v/v) Cap A/Cap B (Isobutyric anhydride/ACN, 1:4 v/v), contact time 2.5 min, charge/push flow rate: 14.4 ml/min, push volume: 1.1 CV, wash volume: 1 CV, wash flow rate: 424 cm/hr. After final detritylation, the CNET protecting group was removed from the phosphodiester linkage with 20% TBA in ACN (5 CV, contact time not less than 45 min, followed by a wash with 4 CV ACN (424 cm/hr)). Cleavage and deprotection was achieved by on-column recirculation with 5 CV of 28-30% ammonia at 50°C for 16 to 24 hrs followed by repeated washing steps of 1 CV water each until a UV reading of less than 40 OD/ml is reached.

The OD was determined by absorption measurement at 260 nm. The oligonucleotide yield (OD/µmol) is given in Table 2.

Anion exchange analysis for determination of the crude oligonucleotide purity was performed on a Waters Alliance HPLC system on a Dionex DNAPac PA200 4x250 mm column at 30°C. The samples are diluted to 6.25 OD/ml in HPLC buffer A (20 mM NaOH), 20 µl of which were injected onto the column and separated at a flow rate of 1 ml/min using a gradient from 25 to 40% HPLC buffer B (20 mM NaOH, 1.5 M NaCl, 40% Methanol) during 5 min followed by a gradient from 40 to 55% buffer B during 35 min. The % full length product is given in Table 2 in comparison to the synthesis of Example 1 with DCI as activator. The data demonstrate that both yield of crude oligonucleotide and purity of FLP are further greatly increased by using ETT as activator. In addition, impurities and synthesis failures such as G10-1n and G10+1n, i.e. compounds comprising one or more G residues less or compounds comprising one or more G residues in addition to the targeted FLP, are further reduced compared to the synthesis with DCI as activator.

**Table 2**

| **Solvent** | **Activator** | **Yield Oligo** [OD/µmol] | **Purity FLP** [% of oligo] | **Content FLP-1n relative to FLP** [%] | **Content LP+1n relative to FLP** [%] |
|---|---|---|---|---|---|
| DMF | DCI | 181 | 56.3 | 5.75 | 0.62 |
| DMF | ETT | 199 | 64.6 | 3.54 | 0.42 |

### EXAMPLE 3

This example describes the synthesis of Example 2 at 105 mmol scale. It demonstrates that the process is scalable to production scale.

This example describes a 105 mmol trityl-off synthesis of SEQ ID NO:1 on a GE OligoProcess oligonucleotide synthesizer using Nittophase High Loaded Unylinker 250 (NPHL 250) as solid synthesis support. 3.85 liter of the synthesis support, swollen in DMF (support density: 9.17 ml/g) were filled into the synthesis column (column diameter 35 cm, column height 4 cm), and after a pre-synthesis wash with ACN (424 cm/hr, 2 CV) the following synthesis cycle was used: (1) detritylation with DCA in toluene (first cycle: 10% DCA in toluene, 62 cm/hr; subsequent cycles: 3% DCA in toluene, 424 cm/hr; 2 CV) followed by ACN wash (2 CV at 200 cm/hr and 2 CV at 424 cm/hr); (2) conditioning with DMF (205 cm/hr, 1.5 CV); (3) coupling (activator: 0.6 M ETT in DMF; deoxynucleoside phosphoramidite (2.0 eq/support): 0.2 M in DMF; charge volume: 2.21 L (52.4% activator volume), charge flow rate: 1.68 L/min; push flow rate: 1.68 L/min; recycle time: 10 min, recycle flow rate: 3.40 L/min) followed by ACN wash (1 CV, 424 cm/hr); (4) pre-oxidation push with 0.5 CV Cap A (NMI/pyridine/ACN, 2:3:5 v/v/v, 2.42 L/min); (5) oxidation with 50 mM 12 (3 eq) in pyridine/water (9:1 v/v), charge volume: 6.30 L, contact time: 2.6 min, charge/push flow rate: 2.42 L/min, oxidation push volume: 1.1 CV, followed by ACN wash (1 CV, 424 cm/hr); (6) pre-capping conditioning with 1.5 CV DMF (205 cm/hr); (7) capping with 0.2 CV Cap A/ACN (1:1 v/v) followed by 0.75 CV of 1:1 (v/v) Cap A/Cap B (Isobutyric anhydride/ACN, 1:4 v/v), contact time 2.5 min, charge/push flow rate: 1.15 L/min, push volume: 1.1 CV, wash volume: 1 CV, wash flow rate: 424 cm/hr. After final detritylation and a final column wash (2 CV ACN, 424 cm/hr), the CNET protecting group was removed from the phosphodiester linkage with 20% TBA in ACN (5 CV, contact time not less than 45 min, followed by a wash with 4 CV ACN (424 cm/hr)). Cleavage and deprotection was achieved by on-column recirculation with 5 CV of 28-30% ammonia at 50°C for 16 to 24 hrs followed by washing with not less than 4 CV water until a UV reading of less than 40 OD/ml is reached.

The OD was determined by absorption measurement at 260 nm.

Anion exchange analysis for determination of the crude oligonucleotide purity was performed on a Waters Alliance HPLC system on a Dionex DNAPac PA200 4x250 mm column at 30°C. The samples are diluted to 6.25 OD/ml in HPLC buffer A (20 mM NaOH), 20 µl of which were injected onto the column and separated at a flow rate of 1 ml/min using a gradient from 25 to 40% HPLC buffer B (20 mM NaOH, 1.5 M NaCl, 40% Methanol) during 5 min followed by a gradient from 40 to 55% buffer B during 35 min.

The crude oligonucleotide yield was 178 OD/µmol (51.6% of max) with a FLP content of 66.7% demonstrating that the preferred process is scalable to production scale.

### EXAMPLE 4

This example describes the purification of crude oligonucleotide synthesized as described in Example 2. It demonstrates that the crude oligonucleotide synthesized using the preferred method can be purified to high purity.

Two batches of crude oligonucleotide synthesized as described in Example 2 were combined to create one feed for the purification process. The purity of FLP in the combined crude oligonucleotide pool was determined with 66.5%. A 7.5 cm diameter column was packed with Source15Q anion exchange matrix to a bed height of 20 cm (compression factor 1) and washed with not less than 3 CV of buffer B (25 mM NaOH, 2 M NaCl) at a flow rate of 100 cm/hr followed by equilibration with not less than 3 CV of buffer A (25 mM NaOH) at a flow rate of 100 cm/hr. The combined crude oligonucleotide was loaded at a concentration of 500 OD/ml at a flow rate of 100 cm/hr followed by a washing step with not less than 1 CV buffer A at 100 cm/hr until the UV signal was back to baseline. For elution, a gradient of 10% buffer B to 44% buffer B was applied over 17 CV at a flow rate of 100 cm/hr, the fraction size was 0.5 CV. Analytical results for mock pools of the fractions analyzed for OD (absorption measurement at 260 nm) and FLP purity (anion exchange HPLC) are shown in Table 3. Mock pools show a high purity of FLP in the range of 94% and high FLP recovery of 71 to 84%.

**Table 3: Chromatography process mock pool data.**

| **Fractions** | **OD recovery [%]** | **FLP [%]** | **FLP recovery [%]** |
|---|---|---|---|
| 17-30 | 58 | 94.3 | 82 |
| 17-31 | 59 | 94.3 | 84 |
| 18-31 | 55 | 94.4 | 78 |
| 19-30 | 50 | 94.9 | 71 |
| 18-30 | 53 | 94.7 | 76 |

Fractions 17 to 31 were pooled for further processing through ultrafiltration/diafiltration (Molecular weight cut-off: 3000) and freeze-drying leading to 6.6 g oligonucleotide (corrected for moisture) with a purity of 93.0% FLP (1% G10-1n and 0.53% G10+1n). The overall process yield was 2.6 g/mmol (19%).

## Claims

1. A method for coupling a nucleoside phosphoramidite during the synthesis of an oligonucleotide to a universal support, to a first nucleoside, or to an extending oligonucleotide, wherein said oligonucleotide comprises a region of 3 or more consecutive guanine monomers, and wherein said method comprising the steps of:
(i) generating a coupling solution, wherein said coupling solution comprises:
(a) said nucleoside phosphoramidite;
(b) an activating reagent; and
(c) one or more solvents, wherein one of said one or more solvents is *N,N-*dimethylformamide (DMF); and
(ii) contacting said coupling solution with said universal support, with said first nucleoside, or with said extending oligonucleotide.

2. The method of claim 1, and wherein the volume of said DMF is equal to or higher than 25%, preferably equal to or higher than 33%, further prefereably equal to or higher than 50%, of the total volume of said one or more solvents.

3. The method of any one of the preceding claim, wherein said one or more solvents comprises, preferably consists of, DMF and acetonitrile, and wherein the ratio (v/v) of said DMF to acetonitrile is between 1:3 and 3:1.

4. The method of any one of the preceding claim, wherein said one or more solvents consists of DMF and acetonitrile, and wherein the ratio (v/v) of said DMF to acetonitrile is 1:1.

5. The method of claim 1, wherein said one or more solvents consists of exactly one solvent, wherein said exactly one solvent is DMF.

6. The method of any one of the preceding claims, wherein said activating reagent is selected from:
(a) 4,5-dicyanoimidazole (DCI);
(b) 5-ethylthio-1*H*-tetrazole (ETT);
(c) 5-benzylthio-1*H*-tetrazole (BTT); or
(d) 5-(3,5-*bis*-trifluoromethyl)phenyl-1*H*-tetrazole (Activator 42).

7. The method of claim 1, wherein said coupling solution comprises, preferably consists of,:
(a) said nucleoside phosphoramidite;
(b) said activating reagent, wherein said activating reagent is is 5-ethylthio-1*H-*tetrazole (ETT)
(c) exactly one solvent, and wherein said exactly one solvent is DMF.

8. The method of any one of the preceding claims, wherein said first nucleoside and/or said extending oligonucleotide is immobilized on a support.

9. The method of any one of the preceding claims, wherein said support is a polystyrene support, wherein said polystyrene support is cross-linked by divinylbenzene.

10. The method of any one of the preceding claims, wherein said support further comprises a linker, wherein said linker is represented by the formula I 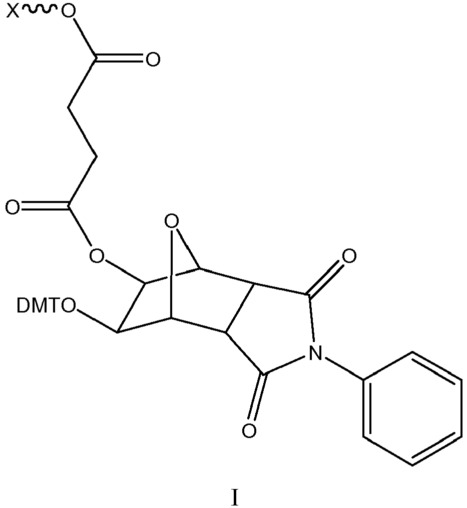 and wherein X represents said support, wherein preferably X represents said polystyrene support cross-linked by divinylbenzene.

11. The method of any one of the preceding claims, wherein said oligonucleotide comprises at least 30 % guanine monomers.

12. The method of any one of the preceding claims, wherein said oligonucleotide comprises a first region of 3 or more consecutive guanine monomers and a second region of 3 or more consecutive guanine monomers, and wherein said first region is located at the 3'-terminus of said oligonucleotide and wherein said second region is located at the 5'-terminus of said oligonucleotide.

13. The method of any one of the preceding claims, wherein said oligonucleotide comprises a nucleotide sequence selected from the group consisting of:
(a) GGGGACGATCGTCGGGGGG (SEQ ID NO:3);
(b) GGGGGACGATCGTCGGGGGG (SEQ ID NO:4);
(c) GGGGGGACGATCGTCGGGGGG (SEQ ID NO:5);
(d) GGGGGGGACGATCGTCGGGGGG (SEQ ID NO:6);
(e) GGGGGGGGACGATCGTCGGGGGGG (SEQ ID NO:7);
(f) GGGGGGGGGACGATCGTCGGGGGGGG (SEQ ID NO:8);
(g) GGGGGGGGGGACGATCGTCGGGGGGGGG (SEQ ID NO:9);
(h) GGGGGGGGGGGACGATCGTCGGGGGGGGGG (SEQ ID NO: 1); and
(i) GGGGGGCGACGACGATCGTCGTCGGGGGGG (SEQ ID NO: 10).

14. The method of any one of the preceding claims, wherein said oligonucleotide consists of SEQ ID NO:1.

15. A method for producing an oligonucleotide, said method comprising
(i) coupling a nucleoside phosphoramidite to a first nucleoside; wherein said coupling comprises the method of any one of claims 1 to 14;
(ii) generating an extending oligonucleotide by oxidizing the product of step (i);
(iii) coupling a nucleoside phosphoramidite to the product of step (ii) after deprotection; wherein said coupling comprises the method of any one of claims 1 to 14;
(iv) generating an extending oligonucleotide by oxidizing the product of step (iii); and
(v) repeating steps (iii) and (iv) until said extending oligonucleotide comprises the sequence of said oligonucleotide.

## Patentansprüche

1. Verfahren zum Koppeln eines Nukleosid-Phosphoramidits während der Synthese eines Oligonukleotids an einen universellen Träger, an ein erstes Nukleosid oder an ein verlängerndes Oligonukleotid, wobei das Oligonukleotid einen Bereich von 3 oder mehr aufeinanderfolgenden Guaninmonomeren umfasst, und wobei das Verfahren die folgenden Schritte umfasst:
(i) Erzeugen einer Kopplungslösung, wobei die Kopplungslösung Folgendes umfasst:
a. das Nukleosid-Phosphoramidit,
b. ein Aktivierungsreagenz und
c. ein oder mehrere Lösungsmittel, wobei eines der einen oder mehreren Lösungsmittel N,N-Dimethylformamid (DMF) ist; und
(ii) In-Kontakt-Bringen der Kopplungslösung mit dem universellen Träger, mit dem ersten Nukleosid oder mit dem verlängernden Oligonukleotid.

2. Verfahren nach Anspruch 1, und wobei das Volumen von DMF gleich oder höher als 25 %, vorzugsweise gleich oder höher als 33 %, ferner vorzugsweise gleich oder höher als 50 % des Gesamtvolumens des einen oder der mehreren Lösungsmittel ausmacht.

3. Verfahren nach einem der vorhergehenden Ansprüche, wobei das eine oder die mehreren Lösungsmittel DMF und Acetonitril umfassen, vorzugsweise aus diesen bestehen, und wobei das Verhältnis (v/v) von DMF zu Acetonitril zwischen 1:3 und 3:1 liegt.

4. Verfahren nach einem der vorhergehenden Ansprüche, wobei das eine oder die mehreren Lösungsmittel aus DMF und Acetonitril bestehen und wobei das Verhältnis (v/v) von DMF zu Acetonitril 1:1 beträgt.

5. Verfahren nach Anspruch 1, wobei das eine oder die mehreren Lösungsmittel aus genau einem Lösungsmittel besteht, wobei das genau eine Lösungsmittel DMF ist.

6. Verfahren nach einem der vorhergehenden Ansprüche, wobei das Aktivierungsreagenz ausgewählt ist aus:
(a) 4,5-Dicyanoimidazol (DCI),
(b) 5-Ethylthio-1*H*-tetrazol (ETT),
(c) 5-Benzylthio-1*H*-tetrazol (BTT) oder
(d) 5-(3,5-*Bis*-trifluormethyl)phenyl-1*H*-tetrazol (Aktivator 42).

7. Verfahren nach Anspruch 1, wobei die Kopplungslösung Folgendes umfasst, vorzugsweise daraus besteht:
(a) das Nukleosid-Phosphoramidit,
(b) das Aktivierungsreagenz, wobei das Aktivierungsreagenz 5-Ethylthio-1*H*-tetrazol (ETT) ist,
(c) genau ein Lösungsmittel, und wobei das genau eine Lösungsmittel DMF ist.

8. Verfahren nach einem der vorhergehenden Ansprüche, wobei das erste Nukleosid und/oder das verlängernde Oligonukleotid auf einem Träger immobilisiert ist.

9. Verfahren nach einem der vorhergehenden Ansprüche, wobei der Träger ein Polystyrolträger ist, wobei der Polystyrolträger durch Divinylbenzol vernetzt ist.

10. Verfahren nach einem der vorhergehenden Ansprüche, wobei der Träger des Weiteren einen Linker umfasst, wobei der Linker durch die Formel I dargestellt ist 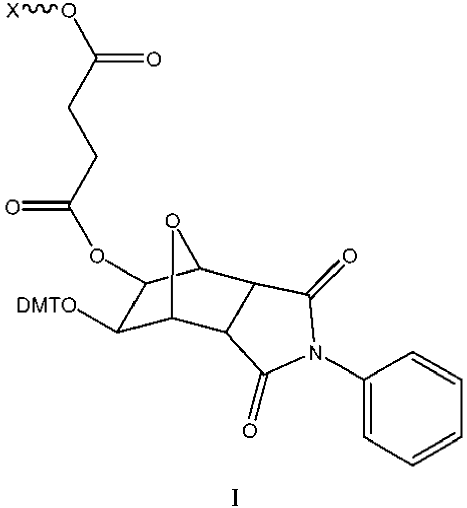 und wobei X den Träger darstellt, wobei vorzugsweise X den Polystyrolträger darstellt, der durch Divinylbenzol vernetzt ist.

11. Verfahren nach einem der vorhergehenden Ansprüche, wobei das Oligonukleotid mindestens 30 % Guaninmonomere umfasst.

12. Verfahren nach einem der vorhergehenden Ansprüche, wobei das Oligonukleotid einen ersten Bereich von 3 oder mehr aufeinanderfolgenden Guaninmonomeren und einen zweiten Bereich von 3 oder mehr aufeinanderfolgenden Guaninmonomeren umfasst, und wobei der erste Bereich am 3'-Terminus des Oligonukleotids liegt und wobei der zweite Bereich am 5'-Terminus des Oligonukleotids liegt.

13. Verfahren nach einem der vorhergehenden Ansprüche, wobei das Oligonukleotid eine Nukleotidsequenz umfasst, ausgewählt aus der Gruppe bestehend aus:
(a) GGGGACGATCGTCGGGGGG (SEQ ID NO: 3);
(b) GGGGGACGATCGTCGGGGGG (SEQ ID NO: 4);
(c) GGGGGGACGATCGTCGGGGGG (SEQ ID NO: 5);
(d) GGGGGGGACGATCGTCGGGGGG (SEQ ID NO: 6);
(e) GGGGGGGGACGATCGTCGGGGGGG (SEQ ID NO: 7);
(f) GGGGGGGGGACGATCGTCGGGGGGGG (SEQ ID NO: 8);
(g) GGGGGGGGGGACGATCGTCGGGGGGGGG (SEQ ID NO: 9);
(h) GGGGGGGGGGGACGATCGTCGGGGGGGGGG (SEQ ID NO: 1); und
(i) GGGGGGCGACGACGATCGTCGTCGGGGGGG (SEQ ID NO: 10).

14. Verfahren nach einem der vorhergehenden Ansprüche, wobei das Oligonukleotid aus SEQ ID NO: 1 besteht.

15. Verfahren zur Herstellung eines Oligonukleotids, wobei das Verfahren Folgendes umfasst:
(i) Koppeln eines Nukleosid-Phosphoramidits an ein erstes Nukleosid, wobei das Koppeln das Verfahren nach einem der Ansprüche 1 bis 14 umfasst;
(ii) Erzeugen eines verlängernden Oligonukleotids durch Oxidieren des Produkts aus Schritt (i);
(iii) Koppeln eines Nukleosid-Phosphoramidits an das Produkt von Schritt (ii) nach Entschützung, wobei das Koppeln das Verfahren nach einem der Ansprüche 1 bis 14 umfasst;
(iv) Erzeugen eines verlängernden Oligonukleotids durch Oxidieren des Produkts aus Schritt (iii); und
(v) Wiederholen der Schritte (iii) und (iv), bis das verlängernde Oligonukleotid die Sequenz des Oligonukleotids umfasst.

## Revendications

1. Procédé pour coupler un nucléoside phosphoramidite pendant la synthèse d'un oligonucléotide sur un support universel, sur un premier nucléoside ou sur un oligonucléotide d'extension, dans lequel ledit oligonucléotide comprend une région d'au moins 3 monomères de guanine consécutifs, et dans lequel ledit procédé comprend les étapes de :
(i) génération d'une solution de couplage, ladite solution de couplage comprenant :
(a) ledit nucléoside phosphoramidite ;
(b) un réactif d'activation ; et
(c) un ou plusieurs solvants, l'un dudit ou desdits solvants étant le *N*,*N*-diméthylformamide (DMF) ; et
(ii) mise en contact de ladite solution de couplage avec ledit support universel, avec ledit premier nucléoside ou avec ledit oligonucléotide d'extension.

2. Procédé selon la revendication 1, et dans lequel le volume dudit DMF est égal ou supérieur à 25 %, de préférence égal ou supérieur à 33 %, de façon encore plus préférée égal ou supérieur à 50 %, du volume total dudit ou desdits solvants.

3. Procédé selon l'une quelconque des revendications précédentes, dans lequel ledit ou lesdits solvants comprennent, de préférence consistent en, DMF et acétonitrile, et dans lequel le rapport (v/v) dudit DMF à l'acétonitrile est entre 1:3 et 3:1.

4. Procédé selon l'une quelconque des revendications précédentes, dans lequel ledit ou lesdits solvants consistent en DMF et acétonitrile, et dans lequel le rapport (v/v) dudit DMF à l'acétonitrile est de 1:1.

5. Procédé selon la revendication 1, dans lequel ledit ou lesdits solvants consistent en exactement un solvant, ledit exactement un solvant étant le DMF.

6. Procédé selon l'une quelconque des revendications précédentes, dans lequel ledit réactif d'activation est choisi parmi :
(a) le 4,5-dicyanoimidazole (DCI) ;
(b) le 5-éthylthio-1*H*-tétrazole (ETT) ;
(c) le 5-benzylthio-1*H*-tétrazole (BTT) ; ou
(d) le 5-(3,5-*bis*-trifluorométhyl)phényl-1*H*-tétrazole (Activateur 42).

7. Procédé selon la revendication 1, dans lequel ladite solution de couplage comprend, de préférence consiste en :
(a) ledit nucléoside phosphoramidite ;
(b) ledit réactif d'activation, ledit réactif d'activation étant le 5-éthylthio-1*H*-tétrazole (ETT) ;
(c) exactement un solvant, et ledit exactement un solvant étant le DMF.

8. Procédé selon l'une quelconque des revendications précédentes, dans lequel ledit premier nucléoside et/ou ledit oligonucléotide d'extension est immobilisé sur un support.

9. Procédé selon l'une quelconque des revendications précédentes, dans lequel ledit support est un support de polystyrène, ledit support de polystyrène étant réticulé par le divinylbenzène.

10. Procédé selon l'une quelconque des revendications précédentes, dans lequel ledit support comprend en outre un lieur, ledit lieur étant représenté par la formule I : 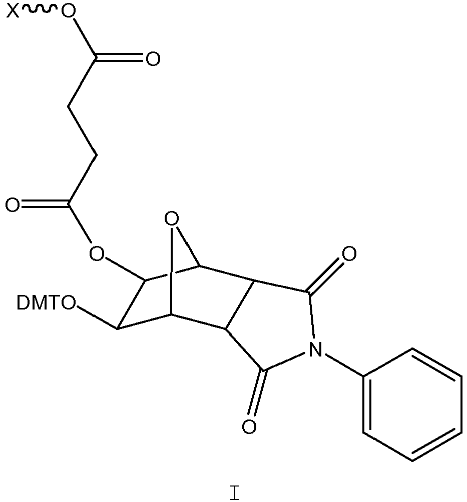 et dans lequel X représente ledit support, dans lequel de préférence X représente ledit support de polystyrène réticulé par le divinylbenzène.

11. Procédé selon l'une quelconque des revendications précédentes, dans lequel ledit oligonucléotide comprend au moins 30 % de monomères de guanine.

12. Procédé selon l'une quelconque des revendications précédentes, dans lequel ledit oligonucléotide comprend une première région d'au moins 3 monomères de guanine consécutifs et une seconde région d'au moins 3 monomères de guanine consécutifs, et dans lequel ladite première région est située à l'extrémité terminale 3' dudit oligonucléotide et dans lequel ladite seconde région est située à l'extrémité terminale 5' dudit oligonucléotide.

13. Procédé selon l'une quelconque des revendications précédentes, dans lequel ledit oligonucléotide comprend une séquence nucléotidique choisie dans le groupe consistant en :
(a) GGGGACGATCGTCGGGGGG (SEQ ID NO:3);
(b) GGGGGACGATCGTCGGGGGG (SEQ ID NO:4);
(c) GGGGGGACGATCGTCGGGGGG (SEQ ID NO:5);
(d) GGGGGGGACGATCGTCGGGGGG (SEQ ID NO:6);
(e) GGGGGGGGACGATCGTCGGGGGGG (SEQ ID NO:7);
(f) GGGGGGGGGACGATCGTCGGGGGGGG (SEQ ID NO:8);
(g) GGGGGGGGGGACGATCGTCGGGGGGGGG (SEQ ID NO:9);
(h) GGGGGGGGGGGACGATCGTCGGGGGGGGGG (SEQ ID NO:1); et
(i) GGGGGGCGACGACGATCGTCGTCGGGGGGG (SEQ ID NO:10).

14. Procédé selon l'une quelconque des revendications précédentes, dans lequel ledit oligonucléotide consiste en SEQ ID NO:1.

15. Procédé de production d'un oligonucléotide, ledit procédé comprenant :
(i) coupler un nucléoside phosphoramidite à un premier nucléoside, ledit couplage comprenant le procédé de l'une quelconque des revendications 1 à 14 ;
(ii) générer un oligonucléotide d'extension par oxydation du produit de l'étape (i) ;
(iii) coupler un nucléoside phosphoramidite au produit de l'étape (ii) après déprotection, ledit couplage comprenant le procédé selon l'une quelconque des revendications 1 à 14 ;
(iv) générer un oligonucléotide d'extension par oxydation du produit de l'étape (iii) ; et
(v) répéter les étapes (iii) et (iv) jusqu'à ce que ledit oligonucléotide d'extension comprenne la séquence dudit oligonucléotide.
